# EUROPEAN PATENT SPECIFICATION

(11) **EP 1 702 598 B1**
(45) Date of publication and mention of the grant of the patent: **03.03.2010**
(21) Application number: 05005695.1
(22) Date of filing: 16.03.2005
(51) Int. Cl.: A61F 13/15, A61F 13/62

(54) **A method of manufacturing pre-fastened absorbent sanitary products**
Verfahren zur Herstellung eines im Voraus befestigten absorbierenden Sanitärartikels
Procédé de fabrication d'articles hygièniques absorbants préfixés

(43) Date of publication of application: 20.09.2006
(73) Proprietor: Fameccanica. Data S.p.A., 66020 Sambuceto di S. Giovanni Teatino (Chieti) (IT)
(72) Inventor: De Angelis, Tonino, 65100 Pescara (IT)
(74) Representative: Bosotti, Luciano

(56) References cited:
- EP-A- 1 523 968
- WO-A-01/87204
- WO-A-01/87207
- WO-A-01/87216
- WO-A-95/27462
- WO-A-02/065961
- US-A1- 2003 018 315

## Description

### Field of the invention

The present invention relates to absorbent sanitary products and has been developed with particular attention paid to its possible application to prefastened absorbent sanitary products that can be worn like a pair of pants.

A typical example of absorbent sanitary products of this type is represented by those baby diapers (nappies) currently referred to as "training pants".

### Description of the related art

For many years now, the solution adopted in a practically uniform way by all the manufacturers of the sector has been that of making these products in the form of elements shaped according to a general hourglass configuration, with a central body, in which there is provided a composite absorbent structure designed to absorb body fluids, and two end parts, a front one and a rear one, which extend laterally. The product is put on, bestowing on it a general U-shaped configuration and bringing the median branch of the central body up to the area where the legs of the user are inserted. The end parts are extended around the waist of the user, connecting the mutually opposed side edges thereof together by means of adhesive labels or stickers, which can normally be re-positioned, or similar fastening elements in such a way as to be able to refasten the product around the body of the user.

These products have been traditionally manufactured and sold in the open condition, i.e., leaving to the person who applies the product the task of setting it around the body of the user and of fastening it according to a general pant-like conformation in the way referred to previously.

In the course of the last few years, there has emerged a renewed interest in diapers of the type commonly referred to as "training pants". These are diapers of the type illustrated, for example, in the document US-A-4 610 680, which are designed for being packaged and sold -- pre-fastened, i.e. in a closed condition. When the pre-fastened product is taken out of the packaging, it has a conformation basically resembling that of a pair of pants. The pre-fastened products is put on by making it slide over the legs of the user according to criteria basically similar to those followed for putting on a pair of pants. In view of the specific use to which the pre-fastened product is to be put, it is then envisaged that the product can be removed without having to slide it again over the legs of the user.

For this purpose, the pre-fastened product can be made in such a way as to be tearable (according to the criteria illustrated, precisely, in US-A-4 610 680), or else by making the pre-fastened product so that it can be opened along the sides of the waist line, i.e., in an area of what may be defined the side flaps (as in the case of diapers of a traditional type, which are packaged and sold in the open condition), for example by envisaging the use of adhesive labels or stickers, sets of buttons, sets of press-studs or, according to a solution that has enjoyed a particular success, by envisaging the use of microhook fastening structures (also referred to as "Velcro fasteners").

The use of such fastening elements must be reconciled with the need, to which reference has already been made previously, to have the training pant produced and packaged pre-fastened, in a closed condition, so as to enable it to be put on like a normal pair of pants.

Various patent documents deal with the problem of applying these fastening elements in the framework of an industrial cycle that can be implemented in a context compatible with the high production rates that are typical of the sector.

In this connection, reference may be made, for instance, to US-A-5 855 574, US-A-6 210 388, US-A-6 409 858, US-A-6 477 628, and US-A-6 461 344.

The above documents, which do not, however, exhaust the entire field on the subject, deal with the problem of the application of the aforesaid fastening elements by drawing particular attention to the functionality of the end product.

At least some of these documents of course take into account the need to make the corresponding products at typical industrial production rates. For this reason, they suggest, according to different modalities, resorting to production processes of a continuous type, in which the products are made starting from the individual component parts, operating preferentially on a continuous chain of products designed for being separated from one another, so as to arrive at the formation of the individual products only in the final stages of the process.

However, above all as regards the application of the aforesaid fastening elements, the solutions described in these prior documents do not take into due account various problems that can assume a considerable importance both at the level of manufacture and at the level of use of the products in question.

In the first place, applying a fastening element (or a part of said element) on a strip, web or chain of sanitary products in the course of fabrication, with the aforesaid strip, web or chain that moves with an even somewhat high linear speed, may expose the aforesaid element, when it is not completely anchored to the strip, web or chain on which it is applied, to phenomena of flapping, which are liable to cause the element itself to be positioned improperly for subsequent operations of treatment, in particular as regards possible operations of cutting.

Furthermore, it is necessary to take into account the fact that the cutting operation for separating the individual sanitary products or the type of connection of the side edges used, can lead to the formation of elements or surfaces of friction which, if not appropriately shielded, may give rise to even rather disagreeable drawbacks, for instance irritation or cutting of the skin, since they directly face the body of the user.

Additionally, it is important to prevent the user (typically, for instance, a baby, perhaps even a very small baby) from possibly opening, with the application of even a modest force, the flaps set on the sides and causing undesirable release of the fastening element, in particular in the case of absorbent products designed to be refastenable.

A number of patent documents specifically deal with the problem of providing sanitary products that are prefastened (i.e. closed), refastenable, and readjustable. Exemplary of these are e.g. US-A-2004/0225271, WO-A-2004/098478, EP-A-0 570 980, US-A-2004/0186451, US-A-2004/0193135, WO-A-03/024377, WO-A-03/024372 and WO-A-03/003923, and WO-A-01/70155.

These prior art arrangements of pre-fastened absorbent sanitary products still fail to provide truly satisfactory results for at least one of a number of reasons, i.e.:
- in certain cases, the "front" and "back" portions of the product are connected at the waist line by tab-like elements that are partly or wholly located within the product; if not appropriately shielded, there may give rise to irritation or cutting of the skin, since they directly face the body of the user;
- in order to be satisfactory, the refastenable and, more to the point, the readjustable feature (i.e. varying the position at which the front and back portions of the waist line of the product are connected) should preferably be achieved by acting on tab-like elements that extend from the back portion of the waist line of the product and are selectively connectable at different positions on the front portion; the opposite arrangement is in fact largely unpractical;
- in still other arrangements, the front and back portions of the waist line of the product are connected via welding areas in the form of lateral "fins" protruding radially from the product; these protruding fins represent a major negative factor in products such as "training pants" that attempt at simulating as closely as possible the appearance of standard pants; additionally, these protruding fins may somewhat facilitate undesired opening of the product by the wearer;
- still another factor to be taken into account lies in that the sanitary products of concern here may not just be baby diapers but also include e.g. incontinence pads for use by adults, essentially in the form of scaled-up versions of baby diapers; the respective packages of these adult products tend to have undesirably large sizes.

The state of the art also includes European patent application 04019792 (EP-A-1523968), that forms part of the state of the art only under the provisions of Art.54(3) EPC, and which was taken as a model for the preamble of claim 1.

### Object and summary of the invention

The purpose of the present invention is to provide an arrangement able to provide a truly satisfactory response to the problems outlined in the foregoing.

According to the present invention, that object is achieved thanks to a process having the characteristics called for specifically in the claims that follow.

The solution described herein leads to the production of a pre-fastened sanitary product wherein, i.a.:
- the front and back portions of the product are connected at the waist line by tab-like elements that are totally located at the outer side of the product, unexposed to the body of the user; consequently such connection/fastening elements are in no way capable of producing irritation or cutting of the skin of the user;
- the refastenable/readjustable feature is achieved by acting on tab-like elements that extend from the back portion of the waist line of the product and are selectively connectable at different positions on the front portion, thus making it possible to adjust the length of the waist line of the product with a view to causing the product to "fit" the wearer's size and habits;
- the front and back portions of the product are connected at the waist line via connection/fastening elements that are totally "flush" (i.e. in the absence of appreciable protrusions) to the outer surface of the product, thus leading the product to have an appearance totally similar to the appearance of standard pants;
- the product as distributed to the public can be folded down to a small size; this leads to a corresponding reduction of the size of the relative package, which is particularly advantageous and appreciated in the case of adults' incontinence pads.

### Brief description of the annexed drawings

The invention will now be described, purely by way of non-limiting example, with reference to the annexed drawings, in which:
- Figure 1 is a general perspective view of a sanitary product as described herein;
- Figure 2 is a cross-sectional view along line II-II of Figure 1;
- Figure 3 is a general perspective view of an alternative embodiment of a sanitary product as described herein;
- Figure 4 is a cross-sectional view along line IV-IV of Figure 3;
- Figure 5 illustrates a closure element adapted to applied on two sanitary products of the type shown in Figure 1 comprised in a chain of products in the course of manufacture;
- Figure 6 illustrates an alternative embodiment of the closure element of Figure 5;
- Figure 7 illustrates a closure element adapted to applied on two sanitary products of the type shown in Figure 3 comprised in a chain of products in the course of manufacture;
- Figure 8 is a schematic representation of a part of the manufacturing process of a sanitary product as shown in Figure 1, and
- Figure 9 shows the resulting product of the process steps shown in Figure 8;
- Figure 10 is a cross-sectional view along line X-X of Figure 9;
- Figure 11 is a cross-sectional view along line XI-XI of Figure 9;
- Figure 12 is representative of how the product of Figure 9 can be packaged; and
- Figure 13 is representative of how the product of Figure 9 can be deployed in order to be worn by the user.

### Detailed description of exemplary embodiments of the invention

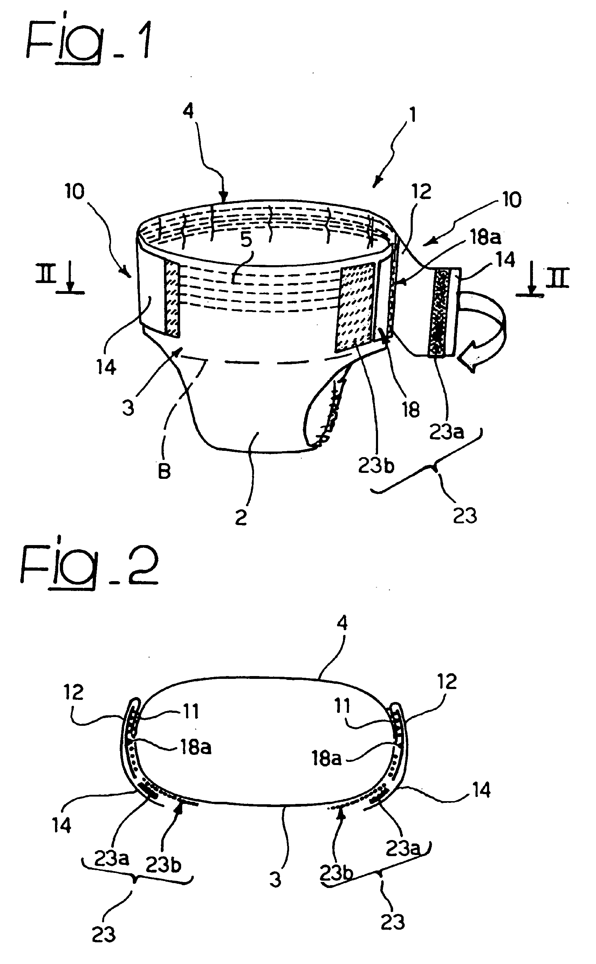

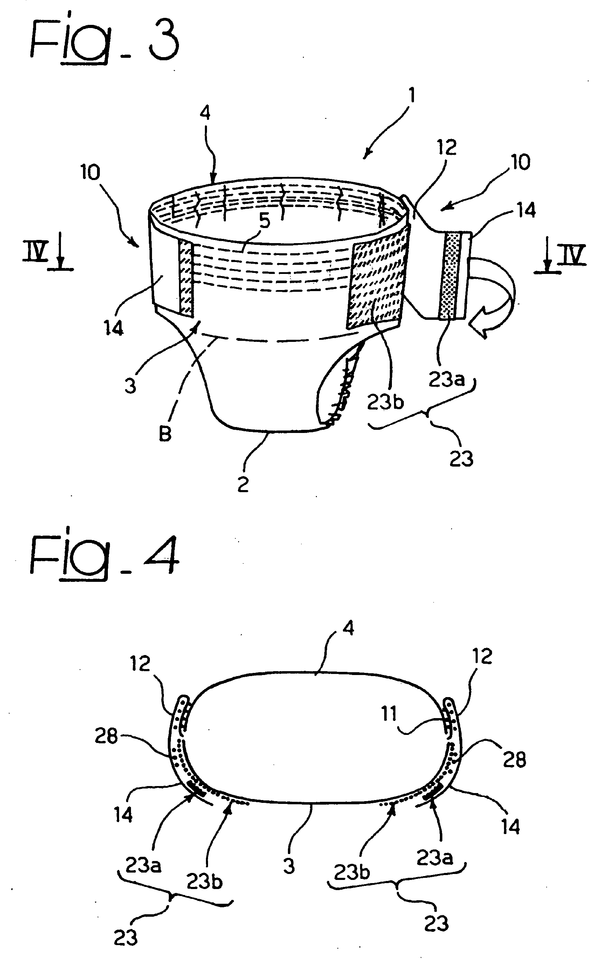

In Figures 1 and 3 pre-fastened absorbent sanitary products of the type currently referred to as a "training pant" are designated 1 as a whole.

Essentially, manufacturing such a pre-fastened product involves producing a pre-formed element or "blank" in the form of a flat element shaped according to a general hourglass configuration, with a central body 2, in which there is provided a composite absorbent structure designed to absorb body fluids, and two end parts, a front one 3 and a rear one 4. The hourglass-shaped flat element is then bestowed a general U-shaped or V-shaped configuration as shown in Figure 1 by bringing the narrower median branch of the central body 2 (currently designated the "crotch portion" of the product) up to the area where the legs of the user will inserted in use.

The front and back parts 3 and 4 are connected at their mutually opposed side edges to form a prfastened, i.e. closed, annular waist portion, whereby the product 1 takes a general form resembling the typical form of standard pants. The product is thus adapted to be worn by a user with the central body 2 extending over and around the "crotch" area of the user, while the waist portion formed by the front and back parts 3 and 4 connected at their mutually opposed side edges surrounds the waist of the wearer.

According to criteria of fabrication that are well known to those of skill in the art, the hourglass-shaped blank from which the product 1 is obtained is typically constituted by:
- a liquid-permeable top or inner sheet (i.e., with respect to the normal conditions of use of the product 1), usually referred to as "topsheet" (TS), on which there can be set a pair of elasticized lateral barriers that extend from one of the borders of the topsheet to the opposite border, and a pair of elastic or elasticized formations set centrally on the ends of said borders, in the direction orthogonal to said elasticized barriers;
- a liquid-impermeable backsheet (BS), i.e., a bottom or outer sheet or laminate (with respect to the normal conditions of use of the product 1), on which there may be set a pair of elastic or elasticized formations that extend from one of the borders of said backsheet to the opposite border; and
- an absorbent structure (AS) to absorb body liquids enclosed i.e. "sandwiched" between the topsheet and the backsheet.

Those of skill in the art are well cognizant that the elements just described constitute a basic structure to which other additional elements can be incorporated according to design principles well known in the art.

For instance, elastic or elasticized formations (see 5 in Figures 1 and 3) are typically arranged centrally on the ends of the borders of the topsheet with the purpose of ensuring adherence of the waist line of the diaper to the abdomen of the user, whilst elastic or elasticized formations (not shown) which extend from one of the borders of the backsheet to the opposite border have the purpose of ensuring adherence of the passages for legs to the legs of the user.

In the finished pre-fastened product 1, the front and back parts 3 and 4 are connected at their mutually opposed side edges to form a closed, annular waist portion of the product 1 by means of label-like connection/fastening assemblies designated 10 having a generally layered structure to be described in greater detail in the following. One such assembly 10 is provided at each side (left-right) of the waist region of the product 1, respectively.

While ensuring that the product 1 - which is intended be of the "pre-fastened" type - as presented to the user (and as originally worn by the wearer), is "closed" around the waist portion as standard pants, the assemblies 10 permit to open the product when worn (e.g. to check whether the wearer is still clean or dry) and then to refasten the product around the body of the user by also adjusting the length of the waist portion to best suit the wearer's size and taste.

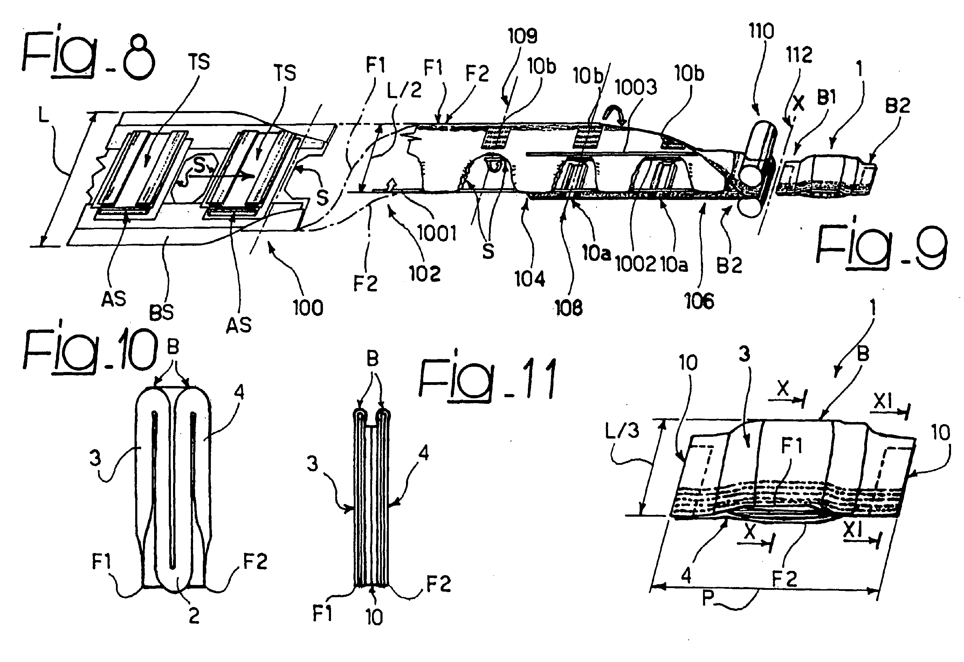

Figure 8 shows hourglass-shaped flat elements ("blanks") of the type described in the foregoing (including an absorbent structure AS sandwiched between a topsheet TS and a backsheet BS) connected in a crosswise arrangement in a chain. Specifically, Figure 8 shows a chain of such hourglass-shaped blanks advancing from left to right and undergoing cascaded shaping and processing operations that lead to the formation of a chain of pants-like products.

As indicated, the general hourglass configuration of the semi-finished pieces or blanks making up such a chain is the result of the generically widened pattern of the lateral end parts with respect to the sides of the central portion (or the area that remains adherent to the body) of each sanitary product. In each product there are present parts S, which are cut out according to a general arched pattern (i.e., the cuts where the legs are inserted); these are the parts that, in the product 1 closed to form a pair of pants, constitute the openings for the legs of the wearer.

The semi-finished pieces are connected together so as to form the chain at the lateral ends of the front and back portions. For this reason, in the chain of semi-finished pieces represented at the top left in Figure 8, the cut-out parts S of the semi-finished pieces adjacent to one another are connected together to form, in the otherwise continuous body of the chain itself, approximately elliptical openings.

In a first folding station (schematically indicated 100, and of any known type for that purpose in the art of manufacturing sanitary products) the outer ends of the backsheet BS are folded inwardly of the chain of semi-finished pieces or blanks to "capture" the corresponding ends of the absorbing structure AS and the topsheet TS at the opposed longitudinal margins F1 and F2 of the chain.

In a further folding station (schematically indicated 102, and again of any known type for that purpose in the art of manufacturing sanitary products) the resulting chain of hourglass-shaped "blanks" undergoes folding to form a U (or V) shape in the central region 2 of the hourglass configuration.

The operation of folding to form a U brings the opposed longitudinal margins F1 and F2 of the chain to overlap one another so as to form (according to criteria described more clearly in what follows) the waist lines of the individual products 1. Simultaneously, the cut-out parts S come to form the passages for the legs in the products themselves.

It will be appreciated that the folding process just described leads the pre-finished products or blanks to have the backsheet BS exposed to the outside of the U-folded product, while the sections of the topsheet TS corresponding to the front and back portions 3, 4 of the product lie against each other in a face-to-face relationship.

In a staggered, interspersed relationship with other processing operations to be described later, the U- (or V-) shaped chain of blanks is subject, at the locations schematically indicated 104 and 106 to two further folding operations.

These folding operations, as the folding operations referred to in the foregoing, gain carried out by means of folding machinery of a type that - per se - is well known in the area of sanitary products. Essentially these folding machines are adapted to operate on a moving chain of advancing products and include a plough-like shaping unit having a rectilinear "ploughshare" folding bar schematically shown and designated 1001, 1002, and 1003 in Figure 8. The central member of the "plough" defines the bend or crease of the fold and the lateral formations that complete the bending movement on both sides of the crease.

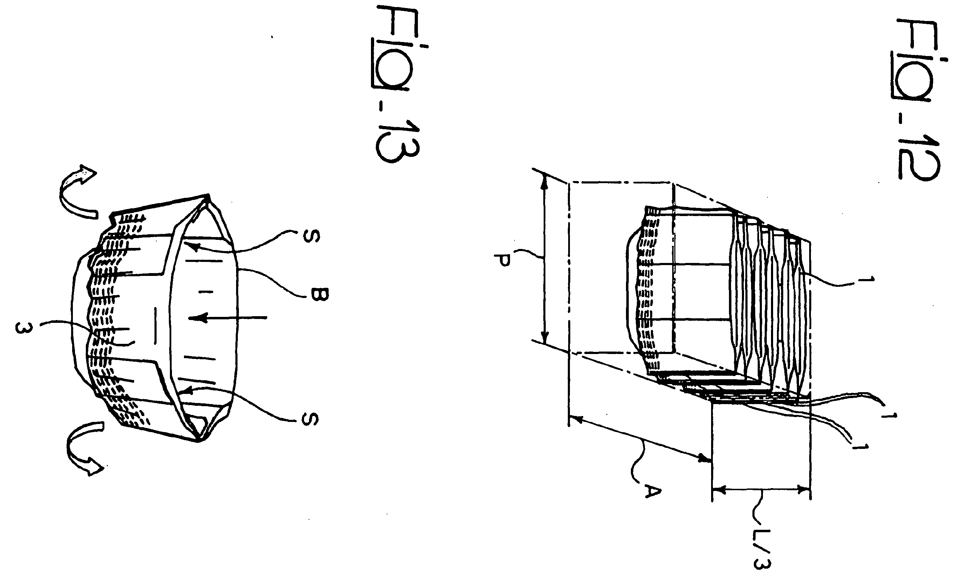

Essentially, while the basic folding operation performed at 102 (folding bar 1001) bestow on the products 1 a general V-like or U-like shape, the purpose of the additional folding operations performed at 104 (folding bar 1002) and 106 (folding bar 1003) is to "open out" the longitudinal margins F1 and F2 and fold them back towards the "crotch" portion (i.e. the portion between the leg openings). This occurs along a folding line B which lies approximately halfway the vertical height of the finished product 1 (see also Figures 1 and 3). The additional folding operations performed at 104 and 106 bestow on the products in the chain an overall M-shape (or an inverted-W shape) as best appreciated in the cross-sectional view of Figure 13.

After the folding operation performed at 104, which leads the - previously downwardly facing - margin F2 to face upwardly, a first element or component 10a of the connecting assembly 10 is applied thereon at 108. Application of the component 10a on the margin F2 is by known means e.g. adhesively or by thermal bonding. A second, complementary element or component 10b of the connecting assembly 10 is similarly applied e.g. at 109 on the upwardly facing margin F1 which is then caused to face downwardly as a result of the bending operation performed at 106.

It will be promptly appreciated that "upwardly" and "downwardly" are used herein in order to facilitate understanding this description and are in no way to be construed in a limiting sense of the scope of the invention: in fact, the bending/shaping processes shown in Figure 13 can be performed with any orientation in space.

As a result of the further bending operation performed at 106, the two complementary elements 10a and 10b are brought into a face-to-face relationship and caused to adhere (i.e. connect) to each other thus "closing" the products 1 along their waste line.

Reference 110 designates a pressing device (including e.g. two counter-rotating rollers) that presses the elements 10a, 10b against each other thus strengthening connection therebetween.

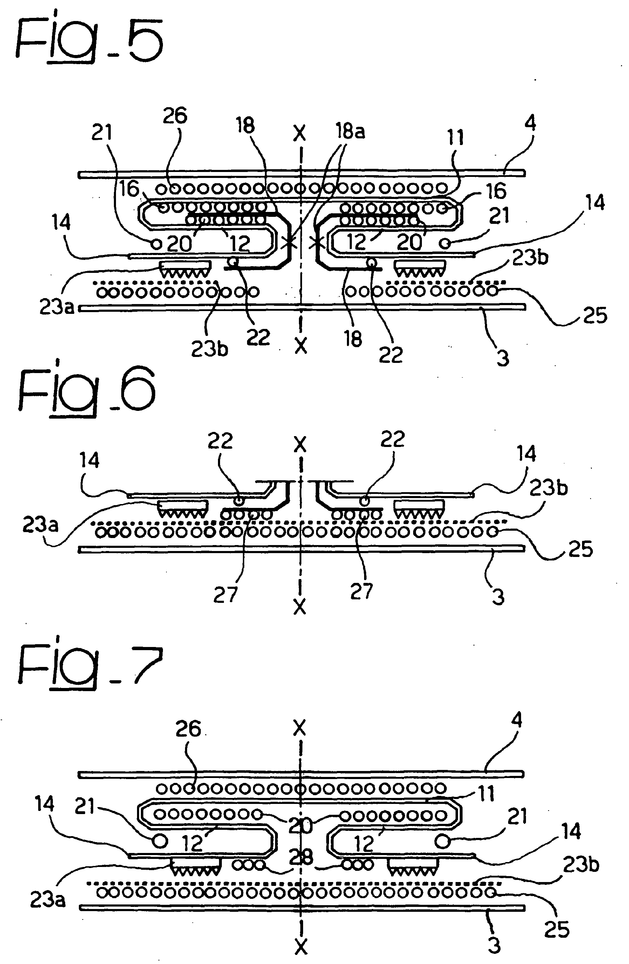

The chain of M-shaped products finally undergoes a cutting operation, implemented for example in a cutting station, designated by 112, of a known type and typically including a rotating-blade cutter. The cutting station 112 operates along a line identified by X-X in Figure 8. The same indication appears - for reasons that will emerge more clearly from what follows - also in Figures 5 to 7.

The cutting action brings about separation of the products 1, which thus assume the character of individual products, which are to be sent on to a packaging station (not illustrated, but of a known type).

The products 1, as emerging from the cutting operation performed at the cutting station 112 have the general appearance shown in Figures 9 to 11, that is they are folded in a M-shaped arrangement with the two end parts 3, 4 spread apart and bent around the respective folding lines B against the crotch portion 2, whereby the two end parts 3, 4 lie against each other at homologous facing side edges with the fastening assemblies 10 sandwiched therebetween. In such an arrangement the crotch portion 2 is located between the front and the back portions 3, 4 "splayed out" i.e. spread apart with the respective topsheet surfaces TS facing outwardly side the product 1 and the respective backsheet BS surfaces facing inwardly of the product and connected by the connecting elements 10a, 10b.

The fastening elements 10a, 10b enable implementation of the function of release and refastening of the product 1, this being according to modalities that enable identification of the product 1 as pre-fastened, releasable and refastenable.

The persons of skill in the art will understand that the characteristics and the modalities of folding the products 1 and application of the fastening elements 10a, 10b (as further detailed in the following) are to a large extent independent of the specific characteristics of embodiment of the various products 1. This applies, in particular, but not exclusively, as regards the structure (or "construction") of the product 1, i.e., as regards characteristics such as the composition of the backsheet BS and of the topsheet TS and of the layers and auxiliary formations normally associated thereto, and so on.

It will be appreciated that in the M- (inverted W-) folded shape shown in Figures 9 to 11 (and 12-13) the product 1 has a length P determined by the "pitch" of the cutting operation performed at 112. The pitch in question essentially corresponds to the "width" of the front and back portions 3 and 4 along the waist line of the wearer, i.e. approximately one half the length of the waist line of the wearer. In fact the length P of the product as packaged is at least slightly smaller than the pitch in question due to the action of the elasticizing members associated with the waist line of the product (see 5 in figure 1) that tend to shorten or contract the product.

Conversely, the "height" of the M-folded product i.e. the distance between the bend lines B and the facing margins F1 and F2, equals a length L3 which is approximately one half of the length L2 of the pre-formed product after the U- or V- bending operation performed at 102 (see Figure 8). The wording "approximately" takes into account the fact that, in the M-folded product, the margins F1 and F2 cannot exactly be aligned with the base of (the crotch portion of) the products due to the space occupied by the v- or U-shape folder (bar 1001).

The length L2 is in turn approximately one half of the total length L of the product measured between the margins F1 and F2 in the product extended flat (see again Figure 8) .

It will be appreciated that in the exemplary arrangement described herein, the M-folded products can be grouped to form stacks adapted to be inserted in respective box-like or bag-like packages, not shown, but of a known type.

As schematically shown in Figure 12, these stacks have a width approximately equal to P, a height approximately equal to L3 and a length A given by the number of products 1 in the stack. Exemplary values for P, L3 and A are 150 mm, 130-140 mm and (in the case of a package including 16 items) 320 mm, respectively.

It will be appreciated that a value of 130-140 mm for L3 leads to a considerable reduction in package size in comparison with standard packages including U- or V-folded products.

The products 1 can be extracted from the package and presented to the wearer in the M-folded configuration shown in Figure 13, i.e. by spreading the front and back sections of the crotch portion so as to enable the wearer to insert his or her legs in the openings S. The folded-cut upper sections of the front and back portions 3 and 4 can then be folded upwardly as schematically shown by the two arrows A and B in Figure 13. In that way the product 1 is restored to its desired pants-like configuration of Figures 1 and 3 following a sort of general "introversion" movement of the M-folded product 1 as packaged.

It will be appreciated that the "introversion" movement just described leads the connection/fastening elements (assemblies) 10 to be brought again at the outer side of the product, which avoids any undesired interaction with the skin of the wearer.

In a possible alternative arrangement (not shown) the "introversion" movement just described can be carried out automatically (by known means) at the end of the production process schematically represented in Figure 8. In that case the movement just described leading to the connection/fastening elements (assemblies) 10 being brought again at the outer side of the product 1 is performed before the product is packaged. The advantages just described in terms of size reduction are not produced if this possible alternative arrangement is resorted to, since the products are packaged just like standard pre-fastened products are currently packaged. An advantage for the user is given in this case as the user is no longer required to perform the "introversion" movement as the connection/fastening elements (assemblies) 10 are already at the outer side of the product as taken from the package.

The method described in connection with Figures 8 to 11 enables the efficient manufacture of hygienic products of the pants-like type that are closed along the waist line by tab-like connection/fastening elements (assemblies) 10 intended to be finally located - outside - the product.

Those of skill in the art will evidently appreciate that use of such a method is in no way limited to connection/fastening elements (assemblies) 10 that:
- are comprised of complementary elements 10a, 10b applied to either of the margins F1 and F2 and then connected as a result of the completion of the M-shape folding process of the products,
- when applied onto the advancing chain of pre-formed product, do in fact include (as better detailed in the following) two symmetrical portions or parts finally intended to be cut along the axis X-X to give rise to two sections that eventually serve as connection/fastening elements for two adjacent products 1 in the chain after these products are separated to produce individual products 1 (cutting station 112 of Figure 8).

Specifically, the solution disclosed in connection with Figures 8 to 12 is adapted for use i.a. in connection with closure/fastening elements that:
- are comprised of a single component applied on either one of the margins F1 or F2 and subsequently attached to the other of the margins F1 or F2 when the M-shape folding process of the products 1 is completed, and/or
- are applied (either as a two-piece assembly as shown in Figure 13 or as a single-piece assembly as mentioned before) as individual items on a respective product 1 at one end of one of the front and/or back portions and, such as, are not subject to any cutting action at 112.

The M-shape folding process exemplified in Figure 8 is applied to a chain of products 1 processed "cross-like", i.e. with the individual hourglass-shaped "blanks" extending crosswise with respect to their direction of advancement. The same process can be applied (by resorting to known means) also to products processed in the "machine direction" that is to a chain of products connected via the end regions of the front and back portions 3 and 4, i.e. with the individual hourglass-shaped "blanks" extending lengthwise with respect to their direction of advancement.

In the exemplary embodiment shown herein, the connection/fastening elements 10 (in the following, the possible partition in two components 10a, 10b will no longer be expressly mentioned for the sake of simplicity) are subject to cutting when the chain of products illustrated in Figure 8 undergoes cutting along the line X-X in the station 112. The elements 10 are also cut along a median or basically median plane, thus being divided into two parts or halves. Of these halves, a first half remains associated to the end B1 of a product 1, by connecting the two corresponding margins F1, F2, and a second half remains associated to the end B2 of the immediately adjacent product, also in this case by connecting the two corresponding margins F1, F2.

If attention is paid precisely to the product 1, which may be seen in the part furthest to the right in Figure 8, it may be understood how the fastening of the waist line of the product in question is ensured, at the end B1, by the first half of an element 10 and, at the end B2, by the second half of another element 10, which has been previously cut in the station 112.

Likewise, in the product 1 that immediately follows the fastening of the waist line is ensured, at the end B2, by the second half of the element 10, the first half of which closes at the end B1 the product D further downstream and, at the end B1 (not, in actual fact, visible in the drawings in so far as this end is shown while still completing the general M-shape folding process of the product), by the second half of another element 10.

Figures 5 to 7 represent three possible embodiments of closure elements in the form of endless strip-like members including two symmetrical portions adapted to be separated by cutting along the axis X-X. Such a strip-like member can be segmented to give rise to to closure elements 10 (possibly including two complementary portions 10a and 10b) adapted to be applied over a chain of products 1 as shown in Figure 8 and then cut (at 112) along the axis X-X to produce two separate portions serving as closure/fastening elements for two adjacent products 1. For additional detail on the possible use of these strip-like closure members reference can be made to European patent application 04019792, already mentioned in the foregoing.

The first embodiment of the closure element 10 represented in Figure 5 corresponds to a base configuration adapted for use in the product 1 shown in Figure 1.

The fastening element 10 represented in Figure 1 includes as a basic component a strip of laminar material, such as a non-woven fabric, for instance of the type commonly referred to as SMS, folded according to a general omega-shaped configuration.

In practice, the strip in question comprises:
- a base branch 11, which extends in a basically symmetrical way around the ideal line X-X along which the element 10 is then to be cut in the operation of separation carried out in the cutting station or cutting unit 112 described previously;
- two intermediate branches 12, each of which is folded on the base branch 11 so as to return towards the line X-X (without reaching it, to prevent undesirable effects of cutting, when the fastening element 1 is divided into two halves along the plane X-X) ; and
- two distal branches 14 turned up again on the intermediate branches 12; typically, the distal branches 14 extend with their end borders approximately at the outer borders of the base branch 11 and of the loops or elbows that connect the intermediate branches 12 with the borders of the base branch 11.

Of course, the choice of making the distal branches 14 extend with their end borders approximately to the outer borders of the base branch 11 is not to be considered in any way imperative. The distal branches 14 can in fact be made so that they are both of equal length, either shorter or longer as compared to the corresponding portions of the base branch 11. The same also applies to the intermediate branches 12.

The whole of the strip making up the branches 11, 12, and 14 or at least a portion thereof in correspondence with the branches 14 can be elastically extendable. Manufacturing and applying such elastic material is well known in the art of sanitary products.

The reference 16 designates a layer of glue designed to render the intermediate branches 12 fixed (i.e. stably applied) to the homologous parts of the base layer or base branch 11. This is preferably via further layers 18, preferably of a non-woven material. The layers 18 are arranged, again in a mirror-like symmetrical way with respect to the axis X-X, to form each a sort of lining extending around the outer surface of the bent portion between one of the intermediate branches 12 and the distal branch 14 extending therefrom.

Extending across each layer 18 along the cusp line thereof, i.e. at the notional line separating the intermediate branch 12 and the distal branch 14 extending therefrom to which the layer 18 serves as a lining, is a weakened line 18a (obtained e.g. by a line of perforations or a line of more easily tearable material e.g. a thinner material). Each layer 18 can thus be selectively torn at the weakened line 18a as better detailed in the following.

Each layer 18 is secured at its opposed ends:
- to the base branch 11, via an extension of the glue layer 16, and to the intermediate branch 12, via a glue layer 20; and
- to the distal branch 14, via a glue layer/strip 22.

The glue layers 16, 20, and 22 may, for instance, be of glue of the hot-melt type or any other type commonly used for the connection of laminar products, such as, precisely, non-woven fabrics in the sanitary-products industry.

The reference number 21 designates provisional-seal formations which can render the intermediate branches 12 fixed to the corresponding distal branches 14. These provisional-seal formations can be made with the application of spots of evergreen or pressure-sensitive glue or by means of welding spots of a thermomechanical type (heat sealing).

The reference numbers 23 designate two releasable connecting formations arranged at the ends of the distal branches 14. The releasable connecting formations 23 are arranged facing outwardly of the general omega-shape of the element 10 and, in the presently preferred embodiment, are comprised of refastenable assemblies of the "microhook" (or "hook-and-loop") type, these being of the kind currently referred to as "Velcro fasteners". Use of different types of releasable connecting formations 23 (e.g. of the adhesive type) serving the same purpose is however within the scope of the invention.

In a particularly preferred embodiment, if "hook-and-loop" formations are used, the "hook" portion 23a is carried (e.g. fixed adhesively) by the distal branch 14, while the "loop" portion 23b is intended to be fixed (again e.g. adhesively at 25) to the chain of products 1, and specifically to the front portions 3 of adjacent products 1 in the chain shown in Figure 8.

A reverse arrangement (i.e. the "loop" portion 23b carried by the distal branch 14 and the "hook" portion 23a fixed to the product 1) is possible. Having the "hook" portion 23a carried by the distal branch 14 is however advantageous since, at least in certain case, the "loop" portion 23b may be simply represented by the surface of the front portion 3 of the product 1, processed in such a way to exhibit the necessary loop structure. Arrangements for providing such a surface with a textile-like structure having loops are known in the art.

Similarly, reference 26 indicates the possibility of adhesively connecting the base branch 11 to the chain of products 1, and specifically to the back portions 4 of adjacent products 1 in the chain shown in Figure 8.

Based on the preceding description of Figure 5, it is easy to understand that partitioning the element designated 10 as a whole into two components such as those designated 10a and 10b in Figure 8 preferably occurs by incorporating to the component indicated 10a all the elements shown in Figure 12 but for the "loop" portions 23b of the releasable formations 23, while the loop portions 23b are incorporated to the component indicated 10b.

In that case, the action of the pressure rollers 110 essentially involves securely connecting the hook portions 23a and the loop portions 23b of the releasable formations 23.

Conversely, if a "one piece" arrangement is preferred for applying the elements 10, a preferred solution is to apply first the whole element 10 onto the front portions 3 of the products 1, and then produce (e.g. at the rollers 110) the attachment of the base branch 11 to the back portions 4 of the products 1.

The main advantage of this arrangement lies in that, after being applied on the front portion 3 of the products 1, the element 10 travels with the product chain a closed, self contained structure, without any free portions likely to extend in an uncontrolled manner and "wave" from the product as it advances.

To sum up, the element 10 comprises two parts that are basically symmetrical with respect to the intermediate plane X-X, each of these parts comprising in turn:
- a base branch 11, which can be connected (at 26) to the back end part 4 of the product; and
- a distal branch 14, which can be connected (at 23) to the front end part 3.

The base branch 11 and the distal branch 14 are connected together according to a general book-like configuration, which opens towards the outside of the element 10. The dorsal parts of said book-like connecting configurations between the base branch 11 and the distal branches 14 are set at a distance apart with respect to said intermediate plane X-X, so that the two basically symmetrical parts comprising the element 10 are connected together by means of the base branch 11 so as to form a single body. This single body can be cut in the intermediate plane X-X in order to separate the two basically symmetrical parts, each of which is able to connect together mutually facing sides of the end parts 3, 4 of a respective absorbent sanitary product 1.

The cross sectional view of Figure 2 highlights the results of applying the process described in connection with Figure 8 by using a closure element 10 of the type shown in Figure 5.

Specifically, Figure 2 is a horizontal sectional view across the the product 1 as worn by a wearer, with the front portion 3 and the back portion 4 of the product shown at the bottom and at the top of the figure, respectively. Additionally, only the reference numerals for the basic parts of the closure/fastening assembly 10 are reproduced in Figure 2.

For easier understanding, the arrangement of parts shown, e.g. at the right side of the product 1 shown in Figure 2 can be thought of as one that has been arrived at by taking the portion of Figure 5 lying on the right hand side of the X-X axis, and by splaying apart and backwards the front and rear portions 3 and 4 of the relative product 1.

As a result, the two portions 3 and 4 are connected by two elements extending in a bridge-like fashion therebetween.

The former of these elements includes the layer 18, having:
- the one end secured to the rear portion 4 via the adhesive layer 26, the base branch 11 and the adhesive layer 16, and
- the other end secured to the front portion 3 via the adhesive layer 25.

More specifically, the said one end of the layer 18 is sandwiched between the base branch 11 and the adjacent intermediate branch 12 and firmly secured therebetween by the adhesive layers 16 and 20. The layer 18 thus forms a bond that firmly connects the front and rear portions 3 and 4 to keep the products 1 in the closed condition typical of pre-fastened products such as "training pants". Such a firm bond can be broken, thus opening the product 1, by tearing the layer in correspondence with the weakened line 18a. The force required to achieve this result can be adjusted by selecting the characteristics (e.g. the thickness) of the layer 18 and/or the characteristics (e.g. the size and number of perforations per unit length) of the weakened line 18a.

The latter of the bridge-like elements referred to in the foregoing includes the combination of the intermediate and distal branches 12 and 14 of the element 10, having:
- the one end (essentially comprised of the intermediate branch 12) secured to the rear portion 4 via the adhesive layer 26, the base branch 11 and the adhesive layers 12 and 16, and
- the other end (essentially comprised of the distal branch 14) removably and readjustably connectable to the front portion 3 via the releasable (e.g. "hook-and-loop") formations 23.

In the product 1, as originally presented to and worn by the wearer in a closed, pants-like configuration, the front and rear portions 3 and 4 are thus firmly connected in correspondence with the waist line of the product 1 by both of the bridge-like elements referred to in the foregoing.

When the need arises of opening the product 1 (e.g. in order to ascertain if the wearer is still clean and dry), the formations 23 are released by separating the complementary portions 23a and 23b. This result can be achieved by exerting moderate force as is the case e.g. of the strip-like hook-and-loop closures that are nowadays conventional in sports footwear.

After the formations 23 are released, the front and rear portions 3 and 4 are connected by the layer 18 only. This can be broken along the weakened line 18a, thus opening the product 1. This operation can be easily performed with moderate effort, while tearing along the weakened line 18a ensures that the two resulting portions of the layer 18, connected to the front portion 3 and back portion 4 of the product 1, are separated precisely along the line 18a, essentially in correspondence with the distal margins of the front portion 3 and back portion 4, without the undesired formation of irregular breakage lines.

After opening the product 1 (e.g. for inspecting it while being worn), the product 1 may be re-closed or re-fastened with the further possibility of adapting the "fit" of the product to the wearer. This result may be achieved by selectively increasing/decreasing the length of the waist line of the product by selectively varying (as shown by way of example at the lower side of Figure 12) the position where the hook element 23a engages the opposed loop element 23b carried or constituted by the outer surface of the front portion 3.

Stated otherwise, in the embodiment shown, the distal branch 14 carrying the element 23a can be fastened onto the front portion 3 at a plurality (typically in the form of a continuum) of different positions, whereby the length of the waist line of the product 1 is selectively varied.

This typically occurs by arranging the hook element 23a closer or farther away from the back portion 4 depending on whether the length of the waist line is sought to be increased or reduced in order to give the product a "tighter" or "looser" fit to the wearer's body.

In the alternative embodiment to which Figure 6 refers (only the lower half-portion of the element 10 is shown in Figure 6, the upper half portion being otherwise identical as shown in Figure 5) the, typically loop-like, components 23b of the releasable formations 23 are extended to the axis X-X to form a single layer adapted to act as a reinforced anchoring layer for adhesive connection (layer 27) of the end of the layer 18 connected to the front portion 3. The resulting single component 23b extends like a bridge connecting the distal branches 14 of the two parts of the element 10 so as to bestow on the element 10 the character of a single body, it being possible for the branch 23b to be cut at the intermediate plane X-X.

Operation of the embodiment of Figure 6 in providing the prefastened, refastenable, and readjustable features of the product 1 is thus essentially is thus essentially identical to the operation of the embodiment of Figure 5 as previously described.

The further alternative embodiment illustrated in Figures 3, 4, and 7 is generally similar to the embodiment represented in Figures 1, 2, and 5 (and 6). For that reason, reference numerals identical to those already appearing in Figures 1, 2, 5, and 6 have been use to designate parts that are identical or equivalent to those already described in the foregoing.

The arrangement illustrated in Figures 3, 4, and 7 can be essentially regarded as a simplified, presently less preferred embodiment obtained from the embodiments of Figures 1, 2, 5 (and 6) by removing the layer 18 and the elements associated therewith.

In brief, in the arrangement of Figures 3, 4, and 7 the role of the layer 18 is played by adhesive connections 28 that anchor the distal branches 14 to the front portion 4. This is typically via the components 23b of the releasable formations 23 extended to the axis X-X (as is the case of Figure 6) to form a single layer adapted to act as a reinforced anchoring layer for adhesive connection (layer 28) of the distal branches 14 to the front portion 3.

It will be appreciated that, while in Figure 7 the adhesive connections 28 are shown arranged "proximally" of the components 23b of the releasable formations 23, the adhesive connections 28 may be alternatively arranged "distally" of the components 23b, namely in the vicinity of the free ends of the distal branches 14.

Additionally, in a possible variant (not shown) the role of the adhesive connections 28 can be played by adhesive (or even just pressure) connections provided along the axis X-X.

The two elements extending in a bridge-like fashion between the two portions 3 and 4 discussed in the foregoing with reference to Figure 5 are in fact incorporated to a single element in the arrangement of Figure 7. That single element is essentially comprised of the intermediate and distal branches 12 and 14; these branches jointly comprise a bridge-like connection assembly extending between the front and rear portions 3 and 4 having (see Figure 7) one end secured to the rear portion 4 via the adhesive layer 26, the base branch 11 and the adhesive layer 16.

The other end of such bridge-like connection assembly is connected to the front portion 3 in a fixed (i.e. tearable, yet non refastenable) manner via the adhesive layer 28, the component 23b acting as a reinforcing layer, and the adhesive layer 25. This arrangement results in a bond that firmly connects the front and rear portions 3 and 4 to keep the products 1 in the closed condition typical of "training pants".

Such a firm bond can be broken, thus opening the product 1, by separating the distal branches 14 from the front portion 3 by "tearing" the adhesive layer 28. Again the force required to achieve this result (typically by means of a traction force exerted on the free ends of the distal branches 14) can be adjusted by selecting - in a known manner - the characteristics of the adhesive layer 28.

The other end of the bridge-like connection assembly referred to in the foregoing is further secured to the front portion 3 in a releasable and refastenable manner (i.e. removably and readjustably) via the releasable (e.g. "hook-and-loop") formations 23.

In the product 1 of Figures 3 and 4, as originally presented to and worn by the wearer in a closed, pants-like configuration, the front and rear portions 3 and 4 are thus firmly connected in correspondence with the waist line of the product 1 by the intermediate and distal branches 12 and 14 that are anchored to the back portion 4 and form a unitary bridge-like connection secured to the front portion 3 in a fixed manner via the adhesive layer 28, the component 23b acting as a reinforcing layer, and the adhesive layer 25.

When the need arises of opening the product 1 (e.g. in order to ascertain if the wearer is still clean and dry), the formations 23 are released by separating the complementary portions 23a and 23b. This result can be again achieved by exerting moderate force as is the case e.g. of the strip-like hook-and-loop closures that are nowadays conventional in sports footwear.

After the formations 23 are released, the front and rear portions 3 and 4 are still connected via the intermediate and distal branches 12 and 14 due to the presence of the adhesive connection represented by the layer 28. This can be broken by pulling the distal branches 14 away from the front portion 3. This operation can be easily performed with moderate effort, while tearing at the layer 28 still ensures a regular separation of the front and back portions 3 and 4, without the undesired formation of irregular breakage lines.

After opening the product 1 (e.g. for inspecting it while being worn), the product 1 may be re-closed or re-fastened with the further possibility of adapting the "fit" of the product to the wearer. This result may be achieved in the same manner as previously described in connection with Figure 12, i.e. by selectively increasing/decreasing the length of the waist line of the product by selectively varying (as shown by way of example at the lower side of Figure 12) the position where the hook element 23a engages the opposed loop element 23b carried or constituted by the outer surface of the front portion 3.

In the embodiment shown in Figures 3 and 4, the distal branch 14 carrying the element 23a can be again fastened onto the front portion 3 at a plurality (typically in the form of a continuum) of different positions, whereby the length of the waist line of the product 1 is selectively varied. This again occurs by arranging the hook element 23a closer or farther away from the back portion 4 depending on whether the length of the waist line is sought to be increased or reduced in order to give the product a "tighter" or "looser" fit to the wearer's body.

The solutions just described lead to the production of a sanitary product wherein, i.a.:
- the front and back portions 3, 4 of the product are connected at the waist line by tab-like elements that (see especially Figures 2 and 4) are totally located at the outer side of the product, unexposed to the body of the user; consequently such connection/fastening elements are in no way capable of producing irritation or cutting of the skin of the user;
- the refastenable/readjustable feature is achieved by acting on the distal branches 14, that is elements that extend from the back portion 4 of the product and are selectively connectable (via the hook-and-loop elements 23) at different positions on the front portion 3;
- the front and back portions 3, 4 of the product are connected at the waist line via connection/fastening elements that are totally "flush" (i.e. in the absence of appreciable protrusions) to the outer surface of the product (see again Figures 2 and 4), thus leading the product to have an appearance totally similar to the appearance of standard pants;
- the product as distributed to the public can be folded down to a small size as shown in Figure 12; this leads to a corresponding reduction of the size of the relative package as exemplified in Figure 12, which is particularly advantageous and appreciated in the case of adults' incontinence pads.

Of course, without prejudice to the principle of the invention, the details of fabrication and the embodiments may vary widely with respect to what is described and illustrated herein, without thereby departing from the scope of the present invention, as defined by the annexed claims.

In particular, it will be appreciated that the details of implementation illustrated herein with reference to each given embodiment, are understood as being freely transferable to one or more of the other embodiments illustrated. Additionally, while the presently preferred embodiments of the invention provide for all the structural and operational features previously disclosed in connections with the assemblies 10 being present in both assemblies currently associated with the product 10, certain less-preferred, alternative embodiments of the invention may provide for such structural and operational features being associated with just one (i.e. "at least one") of the assemblies 10. Furthermore, it will be appreciated that the solutions of connection described herein with reference to mechanisms of adhesive connection can be replaced by equivalent solutions, which envisage, for instance, the use of mechanisms of connection by means of heat sealing and/or ultrasonic sealing.

## Claims

1. A method of producing a pre-fastened absorbent sanitary product (1) to be worn like a pair of pants, said product (1) having an outer side with an outer surface of the product and comprising a two end parts (3, 4) and a crotch portion (2) extending between said two end parts (3, 4), wherein said two end parts (3, 4) have, on opposite sides of the product (1), pairs of homologous facing side edges connected together by respective fastening assemblies (10) so as to define the waist line of the product (1), wherein the method includes the steps of:
- producing flat blanks for said products (1), said blanks having an outer side with an outer surface of the products and comprising two end parts (3, 4) and a crotch portion (2) extending between said two end parts (3, 4), wherein said two end parts (3, 4) have, on opposite sides of the product (1), pairs of homologous facing side edges, the method **characterized in that** it includes the steps of:
- folding (102, 104, 106) said flat blanks for said products (1) in a M-shaped arrangement with said two end parts (3, 4) spread apart and folded along respective folding lines (B) against said crotch portion (2), whereby said two end parts (3, 4) lie against each other at said homologous facing side edges, and
- arranging (108, 109) said fastening assemblies (10) sandwiched between said two end parts (3, 4) lying against each other at said homologous facing side edges, whereby said two end parts (3, 4) are connected together by respective fastening assemblies (10) so as to define the waist line of the product (1), whereby once the product is restored to its pants-like configuration from said M-shaped arrangement said fastening assemblies (10) are located at the outer side of the product (1) flush to said outer surface.

2. The method of claim 1, **characterized in that** said two end parts (3, 4) have respective topsheet (TS) and backsheet (BS) surfaces, the method includes the steps of arranging said two end parts (3, 4) spread apart in said M-shaped folded arrangement with said respective topsheet surfaces facing outwardly of the product (1) and said respective backsheet surfaces facing inwardly of the product (1) and connected by the said fastening assemblies (10).

3. The method of either of claims 1 or claim 2, **characterized in that**, in said M-shaped folded arrangement, said crotch portion (2) is located between said front and the back portions (3, 4).

4. The method of any of claims 1 to 3, **characterized in that** it includes the step of packaging said products (1) folded in said M-shaped folded arrangement.

5. The method of any of claims 1 to 4, **characterized in that** it includes the steps of:
- subjecting said products (1) folded in said M-shaped folded arrangement to an introversion movement leading said fastening assemblies (10) to be brought at the outer side of the products (1) whereby said products (1) are restored to a pants-like configuration; and
- packaging said products (1) restored to said pants-like configuration.

## Patentansprüche

1. Verfahren zum Herstellen eines im voraus befestigten absorbierenden Sanitärartikels (1), der wie ein paar Hosen zu tragen ist, wobei der Artikel (1) eine Außenseite mit einer Außenoberfläche des Artikels hat und zwei Endteile (3, 4) und einen Schrittabschnitt (2), der sich zwischen den beiden Endteilen (3, 4) erstreckt, enthält, wobei die beiden Endteile (3, 4) auf gegenüberliegenden Seiten des Artikels (1) Paare gleichwertiger, einander zugewandter Seitenränder haben, die miteinander durch jeweilige Befestigungsanordnungen (10) verbunden werden, so dass sie eine Taillenlinie des Artikels (1) bilden, wobei das Verfahren folgende Schritte enthält:
- Herstellen flacher Rohlinge für die Artikel (1), wobei die Rohlinge eine Außenseite mit einer Außenoberfläche der Artikel haben und zwei Endteile (3, 4) und einen Schrittabschnitt (2) haben, der sich zwischen den beiden Endteilen (3, 4) erstreckt, wobei die beiden Endteile (3, 4) auf gegenüberliegenden Seiten des Produktes (1) Paare gleichwertiger, einander zugewandter Seitenränder haben, wobei das Verfahren **dadurch gekennzeichnet ist, dass** es folgende Schritte enthält:
- Falten (102, 104, 106) der flachen Rohlinge für die Artikel (1) in einer M-förmigen Anordnung, wobei die beiden Endteile (3, 4) ausgebreitet und entlang entsprechender Faltlinien (B) gegen den Schrittabschnitt (2) gefaltet sind, wobei die beiden Endteile (3, 4) an den gleichwertigen, einander zugewandten Seitenrändern aneinander anliegen, und
- Anordnen (108, 109) der Befestigungsanordnungen (10) sandwichartig zwischen den beiden Endteilen (3, 4), die an den gleichwertigen, einander zugewandten Seitenrändern aneinander anliegen, wobei die beiden Endteile (3, 4) durch jeweilige Befestigungsanordnungen (10) miteinander verbunden werden, so dass sie eine Taillenlinie des Artikels (1) bilden, wobei, sobald der Artikel in seine hosenähnliche Konfiguration aus der M-förmigen Anordnung wiederhergestellt wird, die Befestigungsanordnungen (10), die sich auf der Außenseite des Artikels (1) befinden, mit der Außenoberfläche bündig sind.

2. Verfahren nach Anspruch 1, **dadurch gekennzeichnet, dass** die beiden Endteile (3, 4) jeweilige Deckseiten- (TS-) und Rückseiten- (BS-) Flächen haben und das Verfahren die Schritte enthält, die beiden Endteile (3, 4) ausgebreitet in der M-förmigen Anordnung mit den jeweiligen Deckseitenflächen von dem Artikel (1) nach außen gewandt und die jeweiligen Rückseitenflächen von dem Artikel (1) nach innen gewandt und verbunden durch die Befestigungsanordnungen (10) anzuordnen.

3. Verfahren nach einem der Ansprüche 1 oder 2, **dadurch gekennzeichnet, dass** sich bei der M-förmig gefalteten Anordnung der Schrittabschnitt (2) zwischen den vorderen und hinteren Abschnitten (3, 4) befindet.

4. Verfahren nach einem der Ansprüche 1 bis 3, **dadurch gekennzeichnet, dass** es den Schritt des Verpackens der Artikel (1) im M-förmig gefalteten Zustand enthält.

5. Verfahren nach einem der Ansprüche 1 bis 4, **dadurch gekennzeichnet, dass** es folgende Schritte enthält:
- Unterziehen der Artikel (1), die in der M-förmigen gefalteten Anordnung gefaltet sind, einer Einkrämpelbewegung, die dazu führt, dass die Befestigungsanordnungen (10) auf die Außenseite des Artikels (1) gebracht werden, wodurch die hosenähnliche Konfiguration der Artikel (1) wiederhergestellt wird; und
- Verpacken der Artikel (1) in der wiederhergestellten, hosenähnlichen Konfiguration.

## Revendications

1. Procédé pour produire un produit hygiénique absorbant préfixé (1) destiné à être porté comme une culotte, ledit produit (1) ayant un côté externe avec une surface externe du produit et comprenant deux parties d'extrémité (3, 4) et une partie d'entrejambe (2) s'étendant entre lesdites deux parties d'extrémité (3, 4), dans lequel lesdites deux parties d'extrémité (3, 4) ont, sur les côtés opposés du produit (1), des paires de bords latéraux homologues en vis-à-vis raccordées ensemble par des ensembles de fixation (10) respectifs afin de définir la ligne de taille du produit (1), dans lequel le procédé comprend les étapes consistant à :
produire des ébauches plates pour lesdits produits (1), lesdites ébauches ayant un côté externe avec une surface externe des produits et comprenant deux parties d'extrémité (3, 4) et une partie d'entrejambe (2) s'étendant entre lesdites deux parties d'extrémité (3, 4), dans lequel lesdites deux parties d'extrémité (3, 4) ont, sur les côtés opposés du produit (1), des paires de bords latéraux homologues en vis-à-vis, le procédé étant **caractérisé en ce qu'**il comprend en outre les étapes consistant à :
plier (102, 104, 106) lesdites ébauches plates pour lesdits produits (1) selon un agencement en forme de M avec lesdites deux parties d'extrémité (3, 4) étalées et pliées le long des lignes de pliage (B) respectives contre ladite partie d'entrejambe (2), moyennant quoi lesdites deux parties d'extrémité (3, 4) se trouvent l'une contre l'autre au niveau desdits bords latéraux homologues en vis-à-vis, et
agencer (108, 109) lesdits ensembles de fixation (10) pris en sandwich entre lesdites deux parties d'extrémité (3, 4) se trouvant l'une contre l'autre au niveau desdits bords latéraux homologues en vis-à-vis, moyennant quoi lesdites deux parties d'extrémité (3, 4) sont raccordées ensemble par des ensembles de fixation (10) respectifs afin de définir la ligne de taille du produit (1), moyennant quoi une fois que le produit est revenu dans sa configuration en forme de culotte par rapport audit agencement en forme de M, lesdits ensembles de fixation (10) sont positionnés au niveau du côté externe du produit (1) de niveau par rapport à ladite surface externe.

2. Procédé selon la revendication 1, **caractérisé en ce que** lesdites deux parties d'extrémité (3, 4) ont des surfaces de feuille supérieure (TS) et de feuille arrière (BS) respectives, le procédé comprend les étapes consistant à agencer lesdites deux parties d'extrémité (3, 4) étalées selon ledit agencement plié en forme de M avec lesdites surfaces de feuille supérieure respectives orientées vers l'extérieur du produit (1) et lesdites surfaces de feuille arrière respectives orientées vers l'intérieur du produit (1) et raccordées par lesdits ensemble de fixation (10).

3. Procédé selon l'une quelconque des revendications 1 ou 2, **caractérisé en ce que**, dans ledit agencement plié en forme de M, ladite partie d'entrejambe (2) est positionnée entre lesdites parties avant et arrière (3, 4).

4. Procédé selon l'une quelconque des revendications 1 à 3, **caractérisé en ce qu'**il comprend l'étape consistant à emballer lesdits produits (1) pliés selon ledit agencement plié en forme de M.

5. Procédé selon l'une quelconque des revendications 1 à 4, **caractérisé en ce qu'**il comprend les étapes consistant à :
soumettre lesdits produits (1) pliés selon ledit agencement plié en forme de M à un mouvement d'introversion conduisant lesdits ensembles de fixation (10) à être amenés au niveau du côté externe des produits (1), moyennant quoi lesdits produits (1) sont ramenés à une configuration en forme de culotte ; et
emballer lesdits produits (1) ramenés dans ladite configuration en forme de culotte.
